# EUROPEAN PATENT SPECIFICATION

(11) **EP 3 621 699 B1**
(45) Date of publication and mention of the grant of the patent: **20.01.2021**
(21) Application number: 18707355.6
(22) Date of filing: 23.02.2018
(51) Int. Cl.: A61Q 11/00, A61K 8/39, A61K 8/49, A61K 8/86

(54) **ORAL CARE COMPOSITIONS**
MUNDPFLEGEZUSAMMENSETZUNGEN
COMPOSITIONS DE PROTECTION ORALE

(30) Priority: 12.05.2017 EP 17170919
(43) Date of publication of application: 18.03.2020
(73) Proprietor: Unilever PLC, London, Greater London EC4Y 0DY (GB); Unilever N.V., 3013 AL Rotterdam (NL)
(72) Inventor: JOINER, Andrew, Bebington Wirral Merseyside CH63 3JW (GB); LITTLEWOOD, David, Thomas, Bebington Wirral Merseyside CH63 3JW (GB); PHILPOTTS, Carole, Jane, Bebington Wirral Merseyside CH63 3JW (GB)
(74) Representative: Tansley, Sally Elizabeth
(86) International application number: PCT/EP2018/054570
(87) International publication number: WO 2018/206171

(56) References cited:
- EP-A1- 1 935 395
- WO-A1-2016/099544

## Description

The present invention relates to oral care compositions that enhance the white appearance of teeth.

Compositions comprising pigments that whiten the teeth are disclosed in WO16099544 and WO 150975709.

EP 1 935 395 discloses whitening composition comprising a pigment having a hue angle, *h*, in the CIELAB system of from 220 to 320 degrees and a soluble deposition aid for the pigment.

The object of the invention is to deliver an improved whitening benefit to the teeth.

### Description of the Invention

In a first aspect, the present invention relates to an oral care composition comprising an alkoxylated alcohol based nonionic surfactant, a pigment having a hue angle, h, in the CIELAB system of from 220 to 320 degrees and a water soluble, polymeric deposition aid.

In a second aspect, the invention relates to the use of the composition described above for enhanced whitening of the teeth.

### Detailed Description of the Invention

Compositions according to the invention comprise a pigment should be having a hue angle, *h*, in the CIELAB system of from 220 to 320 degrees most preferably between 250 and 290 degrees. A detailed description of hue angle may be found on p57 of Colour Chemistry 3rd edition by H. Zollinger published by Wiley-VCH.

A pigment is generally understood to be a shade/material which is insoluble in the relevant medium, at the relevant temperature. This is in contrast to dyes which are soluble. In the context of this invention, the "relevant medium" is human saliva, the liquid medium in which the composition is used, at the temperature of the oral cavity during brushing of the teeth, i.e. up to 37°C. As a reasonable approximation, the relevant medium may be considered to be water and the relevant temperature to be 25°C.

Preferably, the pigment is capable of reflecting sufficient light such that the treated tooth is perceivably whiter than its initial colour. Preferably, the pigment is coloured such that its natural colour is within the violet-red to green-blue colour, preferably from violet to blue.

Preferably, the pigment is violet or blue, more preferably one of those listed in the Colour Index International. These pigments are listed as pigment violet 1 through to pigment violet 56 and pigment blue 1 through 83.

The preferred pigment violets are pigment violet 1, 1:1, 1:2, 2, 3, 5:1, 13, 19, 23, 25, 27, 31, 32, 37, 39, 42, 44 and 50.

The preferred pigment blues are pigment blue 1, 2, 9, 10, 14, 15, 15:1, 15:2, 15:3, 15:4, 15:6 16, 18, 19, 24:1, 25, 56, 60, 61, 62 and 66.

Other suitable pigments are pigment ultramarine blue and ultramarine violet.

Preferably, the pigment is Pigment Blue 15, more preferably Pigment Blue 15:1, 15:2, 15:3, 15:4, 15:5 or 15:6, most preferably 15:1.

### Blue covarine is especially preferred

The amount of pigment in the composition is preferably from 0.001 to 0.3%, more preferably from 0.005 to 0.1%, and most preferably from 0.01 to 0.08% by weight. The percentage weights refer to the total amount of active present and exclude any carriers that may be present. The pigment may be uniformly spread throughout the composition or, it may be dispersed in a second phase such as a stripe or other coextruded second phase.

Compositions of the invention comprise a water soluble, polymeric deposition aid. In the context of this invention, a "water soluble" polymeric deposition aid is a material that is soluble in water, typically having a solubility of 0.5% or greater, and more typically 5% or greater by weight, at 25°C. Further, such a material remains soluble following drying - i.e. it can be re-dissolved following drying. Such materials are polymers, but are not film-forming polymers. Water solubility is required in order to avoid build up of the deposition aid on the teeth, something that can also be a particular problem with film-forming polymers.

The soluble polymeric deposition aid enhances deposition of the pigment onto the teeth and thereby enhances the colour change caused by the pigment. More particularly, a deposition agent in accordance with the invention is able to enhance the deposition of a pigment such as Blue Covarine when incorporated in a composition at a level of 0.02% by weight, particularly when the deposition aid is itself incorporated at a level of 1% by weight.

The deposition aid is incorporated into the composition at preferably from 0.01 to 10%, more preferably at from 0.05 to 5%, and most preferably at from 0.1 to 1% by weight.

Deposition aids for use in accordance with the present invention are typically high molecular weight polymers, i.e. polymers having a molecular weight of 200,000 or greater. Preferred deposition aids are Gantrez® type polymers and high molecular weight PEGs. Particularly preferred deposition aids are Gantrez® type polymers and high molecular weight PEGs. Especially preferred deposition aids are Gantrez® type polymers.

Gantrez® type polymers are co-polymers of maleic anhydride with methyl vinylether, in which the anhydride moiety may be in a partially or fully hydrolysed or alcoholysed form. Gantrez® polymers themselves are available from ISP Inc. Suitable Gantrez® polymers are: Gantrez S-95: molecular weight 216,000; free acid; Gantrez S-96: molecular weight 700,000; free acid; Gantrez S-97: molecular weight 1,500,000; free acid; and Gantrez MS-955: molecular weight 1,060,000; calcium/sodium salt. Gantrez® type polymers in which the anhydride moiety is fully hydrolysed are preferred. These may be thought of as co-polymers of maleic acid and methyl vinylether. Particularly preferred co-polymers of maleic acid and methyl vinylether have a molecular weight of 1,000,000 or greater and an especially preferred material is Gantrez S-97.

High molecular weight PEGs are poly(ethyleneglycol) polymers having a molecular weight of 1,000,000 or greater, preferably 2,000,000 or greater. A preferred high molecular weight PEG is Polyox 60K, a polymer having a molecular weight of approximately 2,000,000.

The oral care composition comprises an alkoxylated alcohol based nonionic surfactant preferably in an amount of from 0.2 to 10% by weight based on the total weight of the composition. Preferred are polyethylene glycol ethers of fatty alcohols in particular polyethylene glycol ethers having from 20 to 40 ethylene oxide groups per unit. An example of such a suitable surfactant includes the group of surfactants known as Steareth surfactants such as Steareth 30. More preferably, the level of non-ionic surfactant is from 0.5 to 7 wt% of the total composition, most preferably from 1 to 5 wt% of the total composition.

It is preferred if the weight ratio of nonionic surfactant to polymeric deposition aid is from 5:1 to 40:1, more preferably from 10:1 to 30:1.

If other surfactants are present it is preferred if the alkoxylated alcohol based nonionic surfactant is at least 75wt% of the total weight of the surfactant in the composition. Preferably the ratio of alkoxylated alcohol based nonionic surfactant to other type surfactant is greater the 2:1, more preferably greater than 3:1, most preferably greater than 5:1. Although other surfactants, such as anionic and amphoteric surfactants, may be present it is preferred if they are limited to levels below 0.5 wt% of the total composition preferably less than 0.1 wt% of the total composition. This is particularly the case with anionic surfactants.

Preferably, the oral care composition comprises water, thickener and abrasive. Suitable thickeners include silicas and calcium carbonate. The preferred thickener is silica.

Preferred abrasive materials include silicas, aluminas, calcium carbonates, dicalcium phosphates, calcium pyrophosphates, hydroxyapatites, trimetaphosphates, insoluble hexametaphosphates and so on, including agglomerated particulate abrasive materials, usually in amounts between 3 and 60% by weight of the oral care composition. The most preferred abrasives are calcium carbonate and silica, especially silica.

The oral care compositions according to the invention may comprise further ingredients which are common in the art, such as:
antimicrobial agents, e.g. Triclosan, chlorhexidine, copper, zinc, and stannous salts such as zinc citrate, zinc sulphate, zinc glycinate, sodium zinc citrate and stannous pyrophosphate, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds, such as 2,2' methylenebis-(4-chloro-6-bromophenol);
anti-inflammatory agents such as ibuprofen, flurbiprofen, aspirin, indomethacin etc.;
anti-caries agents such as sodium- and stannous fluoride, aminefluorides, sodium monofluorophosphate, sodium trimeta phosphate and casein;
plaque buffers such as urea, calcium lactate, calcium glycerophosphate and strontium polyacrylates;
vitamins such as Vitamins A, C and E;
plant extracts;
desensitising agents, e.g. potassium citrate, potassium chloride, potassium tartrate, potassium bicarbonate, potassium oxalate, potassium nitrate and strontium salts;
anti-calculus agents, e.g. alkali-metal pyrophosphates, hypophosphite-containing polymers, organic phosphonates and phosphocitrates etc.;
biomolecules, e.g. bacteriocins, antibodies, enzymes, etc.;
flavours, e.g. peppermint and spearmint oils;
proteinaceous materials such as collagen;
preservatives;
opacifying agents;
colouring agents;
pH-adjusting agents;
sweetening agents;
pharmaceutically acceptable carriers, e.g. starch, sucrose, water or water/alcohol systems etc.;
humectants such as glycerol, sorbitol, propyleneglycol, xylitol, lactitol etc.;
binders and thickeners such as sodium carboxymethyl-cellulose, xanthan gum, gum arabic etc. as well as synthetic polymers such as polyacrylates and carboxyvinyl polymers such as Carbopol®;
buffers and salts to buffer the pH and ionic strength of the oral care composition; and
other optional ingredients that may be included are e.g. bleaching agents such as peroxy compounds e.g. potassium peroxydiphosphate, effervescing systems such as sodium bicarbonate/citric acid systems, colour change systems, and so on.

Liposomes may also be used to improve delivery or stability of active ingredients.

The oral care compositions may be in any form common in the art, e.g. toothpaste, gel, mousse, aerosol, chewing gum, lozenge, powder, cream, and may also be formulated into systems for use in dual-compartment type dispensers. Preferred forms are toothpastes.

In the following non-limiting examples, amounts are percentages by weight unless otherwise stated.

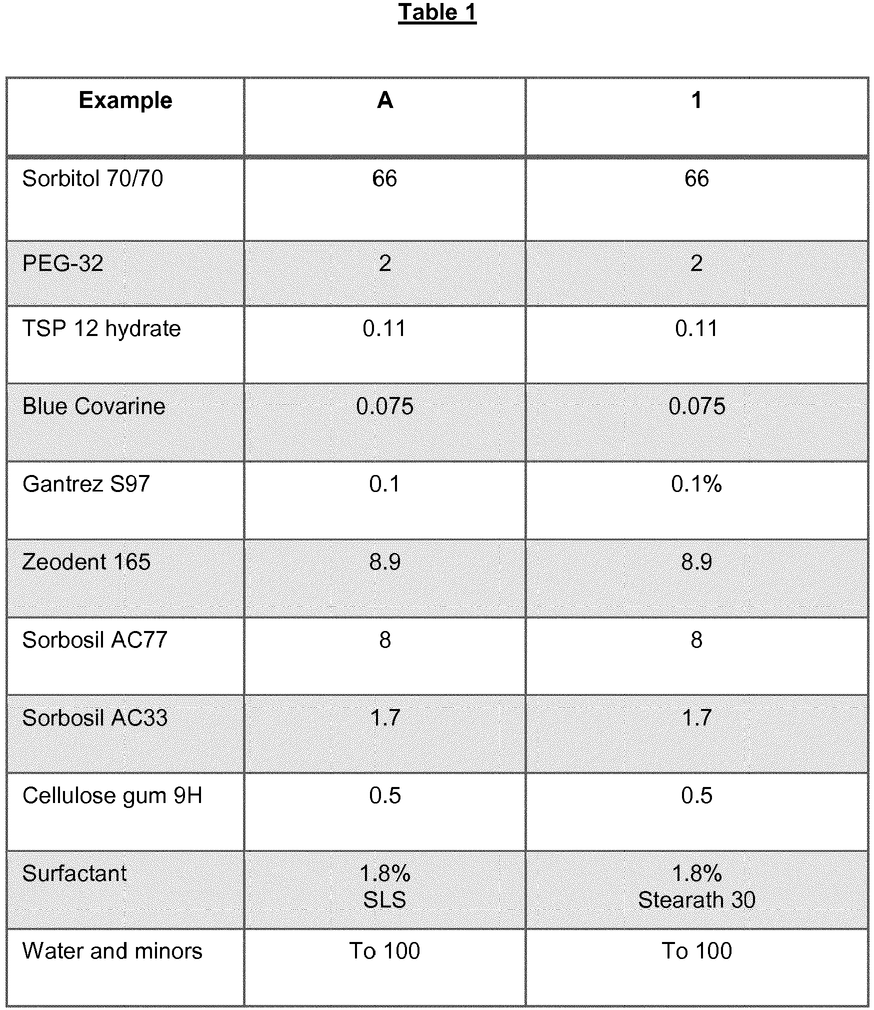

The above formulations were used to whiten teeth as follows. Extracted human incisors and premolars were cleaned with a white toothpaste and placed in sterile saliva for two hours. After rinsing with water, baseline tooth colour was measured using a colorimeter. Tooth specimens were then brushed with a water:toothpaste slurry (2:1) of the test toothpastes for 60 seconds with a toothbrush, rinsed with water and their colour remeasured. Changes in tooth colour were calculated using the WIO tooth whiteness index.

The whitening results are shown in table 2.

**Table 2**

| Example | Change in WIO |
|---|---|
| A | 0.4 |
| 1 | 5.6 |

Formulations with alternative surfactants.

**Table 3**

| **Ingredient** | **Ex. 2** | **Ex. B** | **Ex. C** | **Ex. D** | **Ex. E** |
|---|---|---|---|---|---|
| sorbitol | 69.5 | 69.5 | 69.5 | 69.5 | 69.5 |
| peg-32 | 2 | 2 | 2 | 2 | 2 |
| trisodium phosphate | 0.11 | 0.11 | 0.11 | 0.11 | 0.11 |
| blue covarine | 0.19 | 0.19 | 0.19 | 0.19 | 0.19 |
| gantrez S97 | 1 | 1 | 1 | 1 | 1 |
| zeodent 165 | 4.5 | 4.5 | 4.5 | 4.5 | 4.5 |
| sorbosil AC77 | 8 | 8 | 8 | 8 | 8 |
| sorbosil AC33 | 1.7 | 1.7 | 1.7 | 1.7 | 1.7 |
| cellulose gum 9H | 0.5 | 0.5 | 0.5 | 0.5 | 0.5 |
| Flavour | 1.3 | 1.3 | 1.3 | 1.3 | 1.3 |
| Steareth 30 | 1.8 | 0 | 0 | 0 | 0 |
| Sodium coco sulfate | 0 | 1.8 | 0 | 0 | 0 |
| Sodium methyl cocoyl taurate | 0 | 0 | 1.8 | 0 | 0 |
| Alpha olefin sulphonate | 0 | 0 | 0 | 1.8 | 0 |
| Sodium cocoyl isethionate | 0 | 0 | 0 | 0 | 1.8 |
| Water and minors | To 100 | To 100 | To 100 | To 100 | To 100 |

Examples were evaluated for tooth whitening as previously described. The whitening results are shown in table 4.

**Table 4**

| **Example** | **Change in WIO** |
|---|---|
| 2 | 7.7 |
| B | 3.6 |
| C | 2.6 |
| D | 1.0 |
| E | 1.3 |

## Claims

1. An oral care composition comprising an alkoxylated alcohol based nonionic surfactant, a pigment having a hue angle, h, in the CIELAB system of from 220 to 320 degrees and a water soluble, polymeric deposition aid.

2. An oral care composition according to claim 1 in which the polymeric deposition aid comprises a co-polymers of maleic anhydride with methyl vinylether, in which the anhydride moiety may be in a partially or fully hydrolysed or alcoholysed form.

3. An oral care composition according to any of the preceding claims, **characterised in that** the deposition aid is a polymer having a molecular weight of 200,000 or greater.

4. An oral care composition according to any preceding claim, **characterised in that** the pigment has a hue angle, h, in the CIELAB system of from 250 to 290 degrees.

5. An oral care composition according to any preceding claim in which the pigment comprises blue covarine.

6. An oral care composition according to any preceding claim in which the pigment is present at a level from 0.01 to 0.3% by weight of the total formulation.

7. An oral care composition according to any proceeding claim in which the non-ionic surfactant comprises an ethoxylated non-ionic surfactant.

8. An oral care composition according to any preceding claim in which the non-ionic surfactant comprises an ethoxylated surfactant having a degree of ethoxylation from 20 to 40.

9. An oral care composition according to any preceding claim in which the non-ionic surfactant is steareth 30.

10. An oral care composition according to any preceding claim in which the weight ratio of nonionic surfactant to polymeric deposition aid is from from10:1 to 30:1.

11. An oral care composition according to any preceding claim in which the alkoxylated alcohol based nonionic surfactant is at least 75wt% of the total weight of the surfactant in the composition.

12. An oral care composition according to any of the preceding claims, comprising water, thickener and abrasive.

13. An oral care composition according to claim 12, wherein the thickener comprises silica.

## Patentansprüche

1. Mundpflegezusammensetzung, umfassend ein nichtionisches Tensid auf Basis von alkoxyliertem Alkohol, ein Pigment mit einem Farbtonwinkel h im CIELAB-System von 220 bis 320 Grad und ein wasserlösliches, polymeres Abscheidungshilfsmittel.

2. Mundpflegezusammensetzung nach Anspruch 1, bei der das polymere Abscheidungshilfsmittel ein Copolymer von Maleinsäureanhydrid mit Methylvinylether umfasst, wobei die Anhydrideinheit in einer teilweise oder vollständig hydrolysierten oder alkoholysierten Form vorliegen kann.

3. Mundpflegezusammensetzung nach irgendeinem der vorhergehenden Ansprüche, **dadurch gekennzeichnet, dass** das Abscheidungshilfsmittel ein Polymer mit einem Molekulargewicht von 200.000 oder mehr ist.

4. Mundpflegezusammensetzung nach irgendeinem vorhergehenden Anspruch, **dadurch gekennzeichnet, dass** das Pigment einen Farbtonwinkel h im CIELAB-System von 250 bis 290 Grad aufweist.

5. Mundpflegezusammensetzung nach irgendeinem vorhergehenden Anspruch, bei der das Pigment Blue Covarine umfasst.

6. Mundpflegezusammensetzung nach irgendeinem vorhergehenden Anspruch, in der das Pigment in einer Menge von 0,01 bis 0,3 Gewichts-% der Gesamtformulierung vorliegt.

7. Mundpflegezusammensetzung nach irgendeinem vorhergehenden Anspruch, bei der das nichtionische Tensid ein ethoxyliertes nichtionisches Tensid umfasst.

8. Mundpflegezusammensetzung nach irgendeinem vorhergehenden Anspruch, bei der das nichtionische Tensid ein ethoxyliertes Tensid mit einem Ethoxylierungsgrad von 20 bis 40 umfasst.

9. Mundpflegezusammensetzung nach irgendeinem vorhergehenden Anspruch, in der das nichtionische Tensid Steareth 30 ist.

10. Mundpflegezusammensetzung nach irgendeinem vorhergehenden Anspruch, bei der das Gewichtsverhältnis von nichtionischem Tensid zu polymerem Abscheidungshilfsmittel 10:1 bis 30:1 beträgt.

11. Mundpflegezusammensetzung nach irgendeinem vorhergehenden Anspruch, in der das nichtionische Tensid auf der Basis von alkoxyliertem Alkohol mindestens 75 Gew.-% des Gesamtgewichts des Tensids in der Zusammensetzung ausmacht.

12. Mundpflegezusammensetzung nach irgendeinem der vorhergehenden Ansprüche, umfassend Wasser, Verdickungsmittel und Schleifmittel.

13. Mundpflegezusammensetzung nach Anspruch 12, wobei das Verdickungsmittel Siliciumdioxid umfasst.

## Revendications

1. Composition pour le soin oral comprenant un tensioactif non ionique à base d'alcool alcoxylé, un pigment ayant un angle de teinte, *h*, dans le système CIELAB de 220 à 320 degrés et un agent d'aide au dépôt polymère, soluble dans l'eau.

2. Composition pour le soin oral selon la revendication 1, dans laquelle l'agent d'aide au dépôt polymère comprend un copolymère d'anhydride maléique avec du méthylvinyléther, dans laquelle la moitié anhydride peut être dans une forme partiellement ou complètement hydrolysée ou alcoolisée.

3. Composition pour le soin oral selon l'une quelconque des revendications précédentes, **caractérisée en ce que** l'agent d'aide au dépôt est un polymère ayant une masse moléculaire de 200 000 ou supérieure.

4. Composition pour le soin oral selon l'une quelconque des revendications précédentes, **caractérisée en ce que** le pigment présente un angle de teinte, *h*, dans le système CIELAB de 250 à 290 degrés.

5. Composition pour le soin oral selon l'une quelconque des revendications précédentes, dans laquelle le pigment comprend de la covarine bleue.

6. Composition pour le soin oral selon l'une quelconque des revendications précédentes, dans laquelle le pigment est présent à une teneur de 0,01 à 0,3 % en masse de la formulation totale.

7. Composition pour le soin oral selon l'une quelconque des revendications précédentes, dans laquelle le tensioactif non-ionique comprend un tensioactif non-ionique éthoxylé.

8. Composition pour le soin oral selon l'une quelconque des revendications précédentes, dans laquelle le tensioactif non-ionique comprend un tensioactif éthoxylé ayant un degré d'éthoxylation de 20 à 40.

9. Composition pour le soin oral selon l'une quelconque des revendications précédentes, dans laquelle le tensioactif non-ionique est stéareth 30.

10. Composition pour le soin oral selon l'une quelconque des revendications précédentes, dans laquelle le rapport de masse de tensioactif non-ionique à agent d'aide au dépôt polymère est de 10:1 à 30:1.

11. Composition pour le soin oral selon l'une quelconque des revendications précédentes, dans laquelle le tensioactif non-ionique à base d'alcool alcoxylé représente au moins 75 % en masse de la masse totale du tensioactif dans la composition.

12. Composition pour le soin oral selon l'une quelconque des revendications précédentes, comprenant de l'eau, un épaississant et un abrasif.

13. Composition pour le soin oral selon la revendication 12, dans laquelle l'épaississant comprend de la silice.
